# EUROPEAN PATENT SPECIFICATION

(11) **EP 1 496 864 B1**
(45) Date of publication and mention of the grant of the patent: **21.03.2007**
(21) Application number: 03728552.5
(22) Date of filing: 23.04.2003
(51) Int. Cl.: A61K 9/14

(54) **PHARMACEUTICAL COMPOSITION CONTAINING LAMOTRIGINE PARTICLES OF DEFINED MORPHOLOGY**
PHARMAZEUTISCHE ZUSAMMENSETZUNG ENTHALTEND LAMOTRIGINE-PARTIKEL MIT DEFINIERTER MORPHOLOGIE
COMPOSITION PHARMACEUTIQUE CONTENANT DES PARTICULES DE LAMOTRIGINE DE MORPHOLOGIE DEFINIE

(30) Priority: 23.04.2002 US 374923 P
(43) Date of publication of application: 19.01.2005
(73) Proprietor: TEVA PHARMACEUTICAL INDUSTRIES LTD., 49131 Petah Tiqva (IL)
(72) Inventor: ARONHIME, Judith, 76217 Rehovot (IL); SAMBURSKI, Guy, 42930 Ganot-Hadar (IL)
(74) Representative: Gallagher, Kirk James
(86) International application number: PCT/US2003/013002
(87) International publication number: WO 2003/090693

(56) References cited:
- WO-A-01/22938
- WO-A-96/17611
- WO-A-97/00681
- ES-A- 8 604 019

## Description

### FIELD OF THE INVENTION

The present invention relates to anti-seizure drugs, more particularly to lamotrigine of defined morphology which is used as an adjunct medication in epilepsy therapy, and to methods of preparing pharmaceutical compositions containing such lamotrigine.

### BACKGROUND OF THE INVENTION

Lamotrigine, whose systematic chemical name is 3,5-diamino-6-(2,3-dichlorophenyl)-1,2,4-triazine, exhibits anticonvulsive activity in humans who are prone to seizures. It is approved by the U.S. Food and Drug Administration for use as an adjunct medication in epilepsy therapy.

Lamotrigine is reported to be poorly soluble in water (0.17 mg ml⁻¹) at room temperature and its solubility is not significantly enhanced by changes in pH.

Development of parenteral formulations of lamotrigine has been hampered by its low aqueous solubility. A sterile aqueous solution of a drug is a desirable parenteral administration vehicle, but due to lamotrigine's low solubility, parenteral administration in such a vehicle would necessitate injecting an undesirably large volume of solution. Pharmaceutical formulators have attempted to address use limitations caused by lamotrigine's poor solubility by developing a water-soluble salt of lamotrigine. Unfortunately, salts of lamotrigine with most widely used pharmaceutically benign counterions, such as citrate, tartrate, or maleate, also have low solubility in aqueous solutions.

U.S. Patent No. 4,847,249 discloses a soluble lamotrigine salt with 2-hydroxyethanesulfonic acid (isethionic acid). Unfortunately, isethionic acid is reported to be unstable to prolonged storage. Consequently, it must be generated, e.g. from a stable metal salt, and used quickly to prepare the lamotrigine salt. Problems with the lability of the counter-ion when in solution with tonicity modifiers customarily employed in parenteral formulations further make this salt an inconvenient form in which to administer the drug.

U.S. Patent No. 5,942,510 (the '510 patent) discloses a lyophilized formulation of lamotrigine mesylate and a bulking agent that can be reconstituted with a liquid carrier up to a lamotrigine concentration of 60 mg ml⁻¹. The reconstituted solution is acidic, having a pH from 2.5 to 5. Parenteral administration of acidic solutions can be painful for the patient. However, the '510 patent warns that a pH buffering agent should not be added to either the freeze-dried formulation or the injectable solution.

Aside from the absence of a counterion that significantly improves the solubility of lamotrigine while allowing it to be administered conveniently and without distress to the patient, lamotrigine is not a good candidate for administration as an acid addition salt because it is reported to be acid sensitive.

Physical as well as chemical modifications can affect a compound's solubility properties, such as the crystalline or amorphous state of the compound and particle size. "Solubility" is the concentration of a solution phase at equilibrium with a given solid phase of the dissolved substance. In a compound that is capable of adopting two or more solid phases (crystalline modifications), a more highly soluble crystalline modification of a compound is thermodynamically less stable than a less soluble crystal modification under those same conditions. Thus, providing a thermodynamically less stable modification may be expected to yield a more highly concentrated solution of the compound in equilibrium with the solid phase compound (although kinetic factors may slow the attainment of equilibrium and the less soluble modification may precipitate out of solution).

Particle size reduction is another physical method that was mentioned which may be tried in order to increase a compound's solubility. Particle size reduction is reported to increase the surface area of the solid phase that is in contact with the liquid medium. However, particle size reduction cannot alter the solubility of the compound in a solvent, which is a thermodynamic quantity. It may compensate for a slow rate of dissolution (a kinetic phenomenon) by increasing the amount of solid compound that is available to "react" with the solvent, i.e. to dissolve. Consequently, when a compound is known to have very poor solubility in a liquid, a decrease in particle size alone cannot be predicted to improve solubility because particle size reduction is reported not to affect thermodynamic stability. There is no suggestion in the art that the low solubility of lamotrigine is attributable to anything other than its low intrinsic, thermodynamic solubility in water.

U.S. Patent No. 5,861,179 (the '179 patent) discloses a solid pharmaceutical formulation comprising lamotrigine. The formulation is made of granules having a particle size of 850 µm or less. The granules are prepared by spray granulating lamotrigine or lamotrigine salt with lactose, starch and crystalline cellulose in the presence of polyvinylpyrrolidone as binder. The '179 patent states that the active ingredient and excipients have particle sizes below 200 µm before granulation. The lamotrigine or lamotrigine salt employed as a starting material typically has a particle size of 125 µm or less.

There is a long-felt and unmet need in the art of pharmacology and especially in the field of parenteral formulations for lamotrigine.

### SUMMARY OF THE INVENTION

The present invention provides a plurality of lamotrigine particles having a specific surface area of from about two to about three and a half square meters per gram. More preferably, the lamotrigine particles having a specific surface area of about three square meters per gram.

The present invention provides a plurality of lamotrigine particles having the diameter of all particles in the plurality is equal to or less than about 100 µm; preferably, is equal to or less than about 50 µm; and most preferably, is equal to or less than about 10 µm.

The present invention provides a pharmaceutical composition comprising a plurality of lamotrigine particles having a specific surface area of from about two to about three and a half square meters per gram. More preferably, the lamotrigine particles having a specific surface area of about three square meters per gram.

The present invention provides a pharmaceutical composition of a plurality of lamotrigine particles. The lamotrigine particles have the diameter of all particles in the plurality is equal to or less than about 100 µm; preferably, is equal to or less than about 50 µm; and most preferably, is equal to or less than about 10 µm.

The pharmaceutical composition can be formulated into a wide variety of dosage forms including being used to prepare solutions of lamotrigine for oral or parenteral administration. Preferably, the dosage form is a solid oral dosage, a liquid oral dosage or a liquid parenteral dosage.

The present invention provides a dosage form comprising the pharmaceutical composition of lamotrigine of defined morphology. Preferably, the dosage form is a solid oral dosage form comprising at least one pharmaceutically acceptable excipient.

Preferably, the solid oral dosage form containing a unit dose of from about 100 to about 400 milligrams of lamotrigine.

The present invention provides a liquid oral dosage form comprising a liquid carrier selected from the group consisting of water, vegetable oil, alcohol, polyethylene glycol, propylene glycol and glycerin.

The present invention provides a liquid dosage form of further comprising at least one excipient. Preferably, the liquid parenteral dosage form further comprising a tonicity modifier. More preferably, the tonicity modifier is dextrose. More preferably, the aqueous vehicle and tonicity modifier is a 5% solution of dextrose.

The present invention provides a liquid parenteral dosage form of lamotrigine of defined morphology further comprising at least one excipient selected from the group consisting of dextrose, glycerol, lactose, mannitol, sorbitol, acetate, citrate, tartrate, parabens, 1,6-dialkyl substituted phenols, benzalkonium chloride, benzethonium chloride, benzyl alcohol, sodium benzoate, chlorobutanol, phenethyl alcohol, sodium bisulfite, sodium metabisulfite and tocopherol.

The present invention provides a method of reducing the incidence of seizures in a patient comprising the step of administering the dosage forms of lamotrigine of defined morphology.

The present invention further provides the lamotrigine dosage form to be administered in adjunct with another seizure inhibiting drug.

### DESCRIPTION OF THE PREFERRED EMBODIMENTS

The present invention provides a plurality of lamotrigine particles of defined morphology. "Defined morphology" of the lamotrigine particles is characterized by their specific surface area and particle diameter. In accordance with the invention, the plurality of lamotrigine particles is characterized by having a specific surface area of from about 2.0 to about 3.5 square meters per gram. More preferably, the specific surface area is about 3 square meters per gram. The diameter of all lamotrigine particles in the plurality is equal to or less than about 100 µm; preferably, is equal to or less than about 50 µm; and most preferably, is equal to or less than about 10 µm.

The present invention also provides a pharmaceutical composition comprising a plurality of lamotrigine particles characterized by the specific surface area and particle diameter. The pharmaceutical composition is useful for preparing compressed solid dosage forms, encapsulated free flowing and compressed dosage forms, enteral solutions, suspensions and elixers and parenteral solutions.

The pharmaceutical composition of this invention comprises a plurality of lamotrigine particles. Particles of the plurality will vary in characteristics and the characteristics of no individual or small proportion of the particles will materially affect the advantages afforded by this invention which may include more rapid dissolution and the potential for improved bioavailability. Rather, the characteristics of the pharmaceutical composition are determined from a statistically significant sampling of the composition and measurement of bulk, or average, properties of the sample. Statistically significant measurements include those with a statistical sampling error of about 2% or less.

A "pharmaceutical composition," as used herein, means a medicament for use in treating a mammal that comprises lamotrigine prepared in a manner that is appropriate for administration to a mammal; preferably, a human. A pharmaceutical composition also may contain one or more pharmaceutical excipients that are non-toxic to the mammal intended to be treated when the composition is administered in an amount effective to treat the mammal. A pharmaceutical composition includes feedstocks for preparing pharmaceutical dosage forms such as tablets, capsules, suspensions or solutions.

The "particle diameter" refers to the equivalent spherical diameter as determined by laser light scattering method.

"Specific surface area" is the ratio of the surface area of lamotrigine particles to its unit mass; expressed herein in square meters/gram. The present invention also provides a pharmaceutical formulation comprising a plurality of lamotrigine particles having a specific surface area of from about 2.0 to about 3.5 square meters per gram. More preferably, the specific surface area is about 3 square meters per gram.

Gas adsorption analysis is a preferred method for determining the surface area of a sample of lamotrigine for purposes of this invention since it provides a direct measurement of surface area. It is therefore considered to be more accurate than other methods such as electron microscopy and laser diffraction that rely upon a model to obtain a value for surface area (e.g. equivalent sphere model).

We measured specific particle surface area by gas adsorption using a Coulter^{™} SA3100 Series adsorption analyzer. The specific surface area of a sample of lamotrigine particles was calculated by the multipoint Brunauer, Emmet and Teller (BET) method. Brunauer, S., P. H. Emmett, E. Teller, *J*. *Am. Chem. Soc.,* 1938, *60,* 309-319. The calculations of the surface area of lamotrigine particles and its precise weight would yield specific surface area (usually expressed as square meters/gram). Accurate determination of the specific surface area of the sample depends upon proper sample preparation. Pure samples should be weighed to ±0.001 gram and thoroughly desorbed of adsorbed gases before analysis. This may be done by exposing the sample to high dynamic vacuum (≤0.0001 torr) at about 70°C for thirty minutes. An approximately one gram sample was loaded into a sample tube (9 cc) of the Coulter gas adsorption analyzer and thoroughly degassed. The volume of the manifold was measured with helium gas using a conventional technique. Adsorption data was generated at -77°C using nitrogen gas (P/Po N₂=0.05-0.3) as adsorbate. The instrument's software calculated the moles of adsorbed N₂ (based upon inputted sample mass, manifold volume and sample tube void volume) for ten equilibrium pressures (Ps). In this range, linearity of the BET equation did not deviate significantly (r=0.999).

The number of moles of N₂ adsorbed in a monolayer (nₘₒₙₒ) onto the surfaces of the lamotrigine particles was calculated by application of the least squares method to the BET equation in its linear form y=ax+b wherein the variables y=Ps/n(P₀-Ps) and x= (Ps/P₀) in which n is the number of moles of N₂ adsorbed and condensed onto the sample, nₘₒₙₒ=1/(a+b) and the other variables are as previously defined. The specific surface area of the particles was then routinely calculated.

Pharmaceutical compositions of the invention include those whose lamotrigine particles meet the invention's surface area criteria and whose lamotrigine particles have a particle diameter of 100 µm or less, more preferably about 50 µm or less and most preferably about 10 µm or less. Lamotrigine having these specific surface areas is advantageous for solubility, bioavailability and rate of dissolution.

Yet more preferred pharmaceutical compositions of the invention contain no lamotrigine particles of diameter greater than about 100 µm.

Lamotrigine of defined particle size may be produced by precipitation from appropriate solvents. Particle diameter may be adjusted by customary methods such as cooling, pH adjustment, pouring a concentrated solution into an anti-solvent and/or by coprecipitation so as to obtain a precipitate with the appropriate specific surface area.

Lamotrigine of defined particle diameter may be produced by known methods of particle size reduction starting with crystals, powder aggregates and course powder of either crystalline or amorphous lamotrigine. The principal operations of conventional size reduction are milling of a feedstock material and sorting of the milled material by size.

A fluid energy mill, or micronizer, is an especially preferred type of mill for its ability to produce particles of small size in a narrow size distribution. As those skilled in the art are aware, micronizers use the kinetic energy of collision between particles suspended in a rapidly moving fluid (typically air) stream to cleave the particles. The suspended particles are injected under pressure into a recirculating particle stream. Smaller particles are carried aloft inside the mill and swept into a vent connected to a dust collector. The feedstock may be pre-milled to about 150 to 850 µm.

The most widely practiced method of sorting by particle size involves passing the milled material through a stack of sieves, each with openings of a different size. The sieves are arranged so that the material encounters the sieve having the largest openings first and those particles that pass through the first sieve encounter a second sieve with smaller openings and those that pass through the second sieve may encounter a third sieve, and so forth. Lamotrigine particles also may be separated by particle size using cyclonic or centrifugation techniques.

Sieves are constructed of wire cloth or finely perforated sheet mounted in a frame. Sieves are classified by the size openings in the wire cloth or perforated sheet. The size of the openings in a wire cloth sieve is determined by the diameter of the wire and the tightness of the weave of the wire cloth. The ASTM E11-01 (available through the worldwide web at http://www.astm.org) series of Standard Test Sieves include sieves with 106 µm, 53 µm and 32 µm openings. Particles with a diameter of from about 100 µm to about 50 µm can be separated from a powder with a broader particle size distribution by using a stack of two-sieves with a 106 µm seive above a 53 µm seive. When the material encounters the sieve stack, particles larger than 106 µm are captured on the first sieve. The oversized particles can be remilled to produce more particles within the desired size range. Fines of diameter less than 53 µm would be allowed to pass through the second sieve and also may be recycled, such as by dissolving them and recrystallizing or granulating them to produce a feedstock suitable for size reduction. Particles within the about 100-50 size range would be collected on the second, 53 µm sieve.

Industry has developed different systems for designating sieve sizes. For instance, 106 µm, 53 µm and 32 µm sieves of the ASTM Standard designation system correspond to Nos. 140, 270 and 450 respectively of an alternative ASTM designation system based on the English system. Another distinct system whose designations parallel those of the ASTM Alternative designation system is the Tyler Designation. The Tyler system is sometimes intended to be referred to when a sieve's size is given in "mesh." One hundred and six micrometer (ASTM Alt. No. 140) and 32 µm (ASTM Alt. No. 270) sieves are designated 150 mesh and 270 mesh respectively in the Tyler system. *See, Perry's Chemical Engineers' Handbook, 6th ed.* p. 21-15 (1984). This disclosure uses the ASTM E11-01 Standard Test Sieve designation.

The pharmaceutical composition of this invention may be formulated into a variety of solid and liquid dosage forms for administration to humans and/or animals. The dosage forms include those suitable for enteral (oral, buccal, rectal) administration and parenteral (including subcutaneous, intramuscular, and intravenous) administration. Dosage forms include solid dosage forms like tablets, powders, capsules, suppositories, sachets, troches and losenges as well as liquid solutions, suspensions, syrups and elixirs. The most suitable route in any given case will depend on the nature and severity of the condition being treated and other circumstances that will be assessed by the caregiver.

The pharmaceutical composition may be made into a solid oral dosage form such as a tablet. For making a tablet, it will typically be desirable to include one or more benign pharmaceutical excipients (i.e., pharmaceutical acceptable excipients) in the composition. The pharmaceutical composition of the present invention may contain one or more diluents added to make the tablet larger and, hence, easier for the patient and caregiver to handle. Common diluents are microcrystalline cellulose (e.g. Avicel^{®}), microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylates (e.g. Eudragit^{®}), potassium chloride, powdered cellulose, sodium chloride, sorbitol and talc.

Binders also may be included to help hold the tablet together after compression. Some typical binders are acacia, alginic acid, carbomer (e.g. carbopol), carboxymethylcellulose sodium, dextrin, ethyl cellulose, gelatin, guar gum, hydrogenated vegetable oil, hydroxyethyl cellulose, hydroxypropyl cellulose (e.g. Klucel^{®}), hydroxypropyl methyl cellulose (e.g. Methocel^{®}), liquid glucose, magnesium aluminum silicate, maltodextrin, methylcellulose, polymethacrylates, povidone (e.g. Kollidon^{®}, Plasdone^{®}), pregelatinized starch, sodium alginate and starch.

The tablet may further include a disintegrant to accelerate disintegration of the tablet in the patient=s stomach. Disintegrants include alginic acid, carboxymethylcellulose calcium, carboxymethylcellulose sodium (e.g. Ac-Di-Sol^{®}, Primellose^{®}), colloidal silicon dioxide, croscarmellose sodium, crospovidone (e.g. Kollidon^{®}, Polyplasdone^{®}), guar gum, magnesium aluminum silicate, methyl cellulose, microcrystalline cellulose, polacrilin potassium, powdered cellulose, pregelatinized starch, sodium alginate, sodium starch glycolate (e.g. Explotab^{®}) and starch.

A pharmaceutical composition for tableting may further include glidants, lubricants, flavorings, colorants and other commonly used excipients.

Liquid oral dosage form of the present invention comprise lamotrigine of defined morphology and a liquid carrier such as water, vegetable oil, alcohol, polyethylene glycol, propylene glycol or glycerin, most preferably water.

Liquid oral dosage form may contain emulsifying agents to disperse uniformly throughout the composition the active ingredient or other excipient that has low solubility in the liquid carrier. Emulsifying agents that may be useful in liquid compositions of the present invention include, for example, gelatin, egg yolk, casein, cholesterol, acacia, tragacanth, chondrus, pectin, methyl cellulose, carbomer, cetostearyl alcohol and cetyl alcohol.

Liquid oral dosage form of the present invention may also contain a viscosity enhancing agent to improve the mouth-feel of the product and/or coat the lining of the gastrointestinal tract. Such agents include acacia, alginic acid bentonite, carbomer, carboxymethylcellulose calcium or sodium, cetostearyl alcohol, methyl cellulose, ethylcellulose, gelatin guar gum, hydroxyethyl cellulose, hydroxypropyl cellulose, hydroxypropyl methyl cellulose, maltodextrin, polyvinyl alcohol, povidone, propylene carbonate, propylene glycol alginate, sodium alginate, sodium starch glycolate, starch tragacanth and xanthan gum.

The liquid oral dosage form also may contain sweetening agents, such as sorbitol, saccharin, sodium saccharin, sucrose, aspartame, fructose, mannitol and invert sugar; preservatives and chelating agents such as alcohol, sodium benzoate, butylated hydroxy toluene, butylated hydroxyanisole and ethylenediamine tetraacetic acid; and buffers such as gluconic acid, lactic acid, citric acid or acetic acid, sodium gluconate, sodium lactate, sodium citrate or sodium acetate.

A preferred dosage form of lamotrigine is a pressurized container of the pharmaceutical composition of the invention that is suitable for the administering lamotrigine as an inhalant.

A preferred dosage form of lamotrigine is a sterile solution for parenteral (e.g., intravenous) administration comprising an aqueous vehicle and lamotrigine, wherein the solution is prepared by contacting a pharmaceutical composition in accordance with this invention with a sterile aqueous liquid. The sterile solution may further contain a tonicity modifying agent such as dextrose glycerol, lactose, mannitol, sorbitol and the like to establish and maintain a suitable osmotic pressure in the aqueous phase. An especially preferred aqueous vehicle with tonicity modifier is a 5% aqueous dextrose solution. The solution for injection may further include a buffer such as acetate, citrate and tartrate; an antimicrobial agent such as parabens, 1,6-dialkyl substituted phenols, benzyl alcohol, sodium benzoate, chlorobutanol, phenethyl alcohol and the like; antioxidants like sodium bisulfite, sodium metabisulfite and tocopherol and the like; as well as any conventional additives that do not cause significant decomposition, hydrolysis of the lamotrigine or ion exchange leading to an insoluble precipitate. For the latter purpose, the use of chloride salts and phosphates is in a parenteral formulation is discouraged. Intravenous administration will be by a series of injections or by continuous infusion over an extended period. Administration by injection or other routes of discretely spaced administration will generally be performed at intervals ranging from weekly to once to three times daily.

A preferred unit dose is a vial or septum-sealed bottle containing the injectable solution of the invention. For adjunctive therapy involving oral administration of a solid or liquid dosage form, an orally administered unit dosage contains normally from 0.15 mg kg⁻¹ day⁻¹ to 25 mg kg⁻¹ day⁻¹. The dosage is generally from 100-400 mg day⁻¹ for adult humans given in single or divided does.

Having thus described the present invention with reference to certain preferred embodiments, the present invention is further illustrated by the following example. The example is provided for illustrative purposes only and is not intended to limit in any way the invention which is defined by the claims.

### EXAMPLE 1

Initially, the lamotrigine particles had a particle diameter distribution wherein 50% of the particles had a diameter equal to or less than 250 µm, and about 80% of the particles had a diameter equal to or less than about 500 µm.

These particles were fed into a 300 mm horizontal type air jet mill operating under the following parameters:

| | |
|---|---|
| Air supplied: | Oil free and dried to a dew point of less than 3°C. |
| Air flow rate: | 7 Nm³/min. |
| Air pressure: | 10 bar |
| Feed air pressure: | 6 bar |
| Grinding air pressure: | 4 bar |
| Nozzle angle: | 32.05° |
| Feed rate: | 29 Kg/h |

Particle diameter of lamotrigine was determined by laser diffraction. We determined the diameter of lamotrigine particles using a Malvern^{™} Mastersizer laser diffraction instrument. Samples of lamotrigine were suspended in hexane containing a surfactant (0.07% dioctyl sulfosuccinate sodium salt). The suspensions were mixed and then sonicated for 15 seconds to thoroughly disperse the lamotrigine particles. The dispersion was then recirculated in the flow cell of the Malvern Mastersizer for two minutes before particle diameter measurements were taken.

The micronized product contained a plurality of lamotrigine particles and the diameter of all particles in the plurality was less than 100 µm.

### EXAMPLE 2

The specific surface area of the lamotrigine particles was measured with a Coulter SA 3100, using the multipoint BET technique. About 1 gram of powder was introduced and weighted in a 9 c.c. tube. The experimental parameters of degassing were 70°C, 30 minutes. For the linearity, 10 points were measured, using high sensitivity.

The value of specific surface area for lamotrigine micronized particles was measured to be 3.1-3.3 square meters per gram.

## Claims

1. A plurality of lamotrigine particles having a specific surface area of from two to three and a half square meters per gram.

2. The plurality of lamotrigine particles of claim 1 having a specific surface area of about three square meters per gram.

3. The plurality of lamotrigine particles of claim 1 wherein the diameter of all particles in the plurality is equal to or less than 100 µm.

4. The plurality of lamotrigine particles of claim 3 wherein the diameter of all particles in the plurality is equal to or less than 50 µm.

5. The plurality of lamotrigine particles of claim 4 wherein the diameter of all particles in the plurality is equal to or less than 10 µm.

6. A pharmaceutical composition comprising a plurality of lamotrigine particles having a specific surface area of from two to three and a half square meters per gram.

7. The pharmaceutical composition of claim 6 having a specific surface area of about three square meters per gram.

8. The pharmaceutical composition of claim 6 wherein the diameter of all particles in the plurality is equal to or less than 100 µm.

9. The pharmaceutical composition of claim 8 wherein the diameter of all particles in the plurality is equal to or less than 50 µm.

10. The pharmaceutical composition of claim 9 wherein the diameter of all particles in the plurality is equal to or less than 10 µm.

11. A dosage form comprising the pharmaceutical composition of any of claims 6 to 10.

12. The dosage form of claim 11 that is a solid oral dosage.

13. The solid oral dosage of claim 12 wherein the pharmaceutical composition comprises at least one pharmaceutically acceptable excipient.

14. The solid oral dosage form of claim 13 wherein the pharmaceutically acceptable excipient is selected from the group consisting of microcrystalline cellulose, microfine cellulose, lactose, starch, pregelatinized starch, calcium carbonate, calcium sulfate, sugar, dextrates, dextrin, dextrose, dibasic calcium phosphate dihydrate, tribasic calcium phosphate, kaolin, magnesium carbonate, magnesium oxide, maltodextrin, mannitol, polymethacrylate, potassium chloride, powdered cellulose, sodium chloride, sorbitol, talc, acacia, alginic acid, carbomer, carboxymethylcellulose sodium, dextrin, ethyl cellulose, gelatin, guar gum, hydrogenated vegetable oil, hydroxyethyl cellulose, hydroxypropyl cellulose, hydroxypropyl methylcellulose, liquid glucose, magnesium aluminum silicate, maltodextrin, methylcellulose, polymethacrylates, povidone, pregelatinized starch, sodium alginate, starch, alginic acid, carboxymethyl cellulose calcium, colloidal silicon dioxide, croscarmellose sodium, crospovidone, guar gum, magnesium aluminum silicate, methyl cellulose, polacrilin potassium, powdered cellulose, pregelatinized starch, sodium alginate and sodium starch glycolate.

15. The solid oral dosage form of any of claims 12 to 14 containing a unit dose of from about 100 to about 400 milligrams of lamotrigine.

16. The dosage form of claim 11 that is a liquid oral dosage.

17. The liquid oral dosage of claim 16 wherein the liquid oral dosage comprises a liquid carrier selected from the group consisting of water, vegetable oil, alcohol, polyethylene glycol, propylene glycol and glycerin.

18. The liquid oral dosage of claim 17 wherein the liquid carrier is water.

19. The liquid oral dosage of any of claims 16 to 18 further comprising at least one excipient selected from the group consisting of gelatin, egg yolk, casein, cholesterol, acacia, tragacanth, chondrus, pectin, methyl cellulose, carbomer, cetostearyl alcohol, cetyl alcohol, alginic acid bentonite, carbomer, carboxymethylcellulose calcium or sodium, ethylcellulose, gelatin guar gum, hydroxyethyl cellulose, hydroxypropyl cellulose, hydroxypropyl methylcellulose, maltodextrin, polyvinyl alcohol, povidone, propylene carbonate, propylene glycol alginate, sodium alginate, sodium starch glycolate, starch tragacanth, xanthan gum, sorbitol, saccharin, sodium saccharin, sucrose, aspartame, fructose, mannitol, invert sugar; ethyl alcohol, sodium benzoate, butylated hydroxy toluene, butylated hydroxyanisole, ethylenediamine tetraacetic acid, guconic acid, lactic acid, citric acid, acetic acid, sodium guconate, sodium lactate, sodium citrate and sodium acetate.

20. The dosage form of claim 11 that is a liquid parenteral dosage.

21. The liquid parenteral dosage of claim 20 further comprising a tonicity modifier.

22. The liquid parenteral dosage of claim 21 wherein the tonicity modifier is dextrose.

23. The liquid parenteral dosage of claim 22 wherein the dextrose is a 5% solution of dextrose.

24. The liquid parenteral dosage of any of claims 20 to 23 further comprising at least one excipient selected from the group consisting of dextrose, glycerol, lactose, mannitol, sorbitol, acetate, citrate, tartrate, parabens, 1, 6-dialkyl substituted phenols, benzalkonium chloride, benzethonium chloride, benzyl alcohol, sodium benzoate, chlorobutanol, phenethyl alcohol, sodium bisulfite, sodium metabisulfite and tocopherol.

25. Use of a dosage form of any of claims 12 to 24 in the manufacture of a medicament for reducing the incidence of seizures.

26. The use of claim 25 wherein the dosage form is administered in adjunct with another seizure inhibiting drug.

## Patentansprüche

1. Mehrzahl von Lamotrigin-Teilchen mit einer spezifischen Oberfläche von zwei bis dreieinhalb Quadratmetern pro Gramm.

2. Mehrzahl von Lamotrigin-Teilchen nach Anspruch 1 mit einer spezifischen Oberfläche von etwa drei Quadratmetern pro Gramm.

3. Mehrzahl von Lamotrigin-Teilchen nach Anspruch 1, wobei der Durchmesser aller Teilchen in der Mehrzahl gleich oder kleiner als 100 µm ist.

4. Mehrzahl von Lamotrigin-Teilchen nach Anspruch 3, wobei der Durchmesser aller Teilchen in der Mehrzahl gleich oder kleiner als 50 µm ist.

5. Mehrzahl von Lamotrigin-Teilchen nach Anspruch 4, wobei der Durchmesser aller Teilchen in der Mehrzahl gleich oder kleiner als 10 µm ist.

6. Pharmazeutische Zusammensetzung, umfassend eine Mehrzahl von Lamotrigin-Teilchen mit einer spezifischen Oberfläche von zwei bis dreieinhalb Quadratmetern pro Gramm.

7. Pharmazeutische Zusammensetzung nach Anspruch 6 mit einer spezifischen Oberfläche von etwa drei Quadratmetern pro Gramm.

8. Pharmazeutische Zusammensetzung nach Anspruch 6, wobei der Durchmesser aller Teilchen in der Mehrzahl gleich oder kleiner als 100 µm ist.

9. Pharmazeutische Zusammensetzung nach Anspruch 8, wobei der Durchmesser aller Teilchen in der Mehrzahl gleich oder kleiner als 50 µm ist.

10. Pharmazeutische Zusammensetzung nach Anspruch 9, wobei der Durchmesser aller Teilchen in der Mehrzahl gleich oder kleiner als 10 µm ist.

11. Dosierungsform, umfassend die pharmazeutische Zusammensetzung nach einem der Ansprüche 6 bis 10.

12. Dosierungsform nach Anspruch 11, welche eine feste orale Dosierungsform ist.

13. Feste orale Dosierungsform nach Anspruch 12, wobei die pharmazeutische Zusammensetzung wenigstens einen pharmazeutisch verträglichen Hilfsstoff umfaßt.

14. Feste orale Dosierungsform nach Anspruch 13, wobei der pharmazeutisch verträgliche Hilfsstoff aus der Gruppe ausgewählt ist, bestehend aus mikrokristalliner Zellulose, mikrofeiner Zellulose, Lactose, Stärke, vorgelatinierter Stärke, Calciumcarbonat, Calciumsulfat, Zucker, Dextraten, Dextrin, Dextrose, zweiwertigem Calciumphosphatdihydrat, dreiwertigem Calciumphosphat, Kaolin, Magnesiumcarbonat, Magnesiumoxid, Maltodextrin, Mannitol, Polymethacrylat, Kaliumchlorid, pulverförmiger Zellulose, Natriumchlorid, Sorbitol, Talk, Akaziengummi, Alginsäure, Carbomer, Carboxymethylzellulosenatrium, Dextrin, Ethylzellulose, Gelatine, Guargummi, hydriertem Pflanzenöl, Hydroxyethylzellulose, Hydroxypropylzellulose, Hydroxypropylmethylzellulose, flüssiger Glucose, Magnesiumaluminiumsilicat, Maltodextrin, Methylzellulose, Polymethacrylaten, Povidon, vorgelatinierter Stärke, Natriumalginat, Stärke, Alginsäure, Carboxymethylzellulosecalcium, kolloidalem Siliciumdioxid, Croscarmellosenatrium, Crospovidon, Guargummi, Magnesiumaluminiumsilicat, Methylzellulose, Polacrilinkalium, pulverförmiger Zellulose, vorgelatinierter Stärke, Natriumalginat und Natriumstärkeglycolat.

15. Feste orale Dosierungsform nach einem der Ansprüche 12 bis 14, welche eine Dosierungseinheit von etwa 100 bis etwa 400 Milligramm an Lamotrigin enthält.

16. Dosierungsform nach Anspruch 11, welche eine flüssige orale Dosierungsform ist.

17. Flüssige orale Dosierungsform nach Anspruch 6, wobei die flüssige orale Dosierungsform einen flüssigen Träger umfaßt, ausgewählt aus der Gruppe, bestehend aus Wasser, Pflanzenöl, Alkohol, Polyethylenglycol, Polypropylenglycol und Glycerin.

18. Flüssige orale Dosierungsform nach Anspruch 17, wobei der flüssige Träger Wasser ist.

19. Flüssige orale Dosierungsform nach einem der Ansprüche 16 bis 18, welche weiterhin wenigstens einen Hilfsstoff umfaßt, ausgewählt aus der Gruppe, bestehend aus Gelatine, Eigelb, Kasein, Cholesterol, Akaziengummi, Tragantgummi, Chondrus, Pektin, Methylzellulose, Carbomer, Cetostearylalkohol, Cetylalkohol, Alginsäurebentonit, Carbomer, Carboxymethylzellulosecalcium oder -natrium, Ethylzellulose, Gelatine, Guargummi, Hydroxyethylzellulose, Hydroxypropylzellulose, Hydroxypropylmethylzellulose, Maltodextrin, Polyvinylalkohol, Povidon, Propylencarbonat, Propylenglycolalginat, Natriumalginat, Natriumstärkeglycolat, Stärketragant, Xanthangummi, Sorbitol, Saccharin, Natriumsaccharin, Saccharose, Aspartam, Fructose, Mannitol, Invertzucker, Ethylalkohol, Natriumbenzoat, butyliertem Hydroxytoluen, butyliertem Hydroxyanisol, Ethylendiamintetraessigsäure Gluconsäure, Milchsäure, Zitronensäure, Essigsäure, Natriumgluconat, Natriumlactat, Natriumcitrat und Natriumacetat.

20. Dosierungsform nach Anspruch 11, welche eine flüssige parenterale Dosierungsform ist.

21. Flüssige parenterale Dosierungsform nach Anspruch 20, welche weiterhin einen Tonizitätsmodifizierer umfaßt.

22. Flüssige parenterale Dosierungsform nach Anspruch 21, wobei der Tonizitätsmodifizierer Dextrose ist.

23. Flüssige parenterale Dosierungsform nach Anspruch 22, wobei die Dextrose eine 5%-ige Lösung von Dextrose ist.

24. Flüssige parenterale Dosierungsform nach einem der Ansprüche 20 bis 23, welche weiterhin wenigstens einen Hilfsstoff umfaßt, ausgewählt aus der Gruppe, bestehend aus Dextrose, Glycerol, Lactose, Mannitol, Sorbitol, Acetat, Citrat, Tartrat, Parabenen, 1,6-Dialkyl-substituierten Phenolen, Benzalkoniumchlorid, Benzethoniumchlorid, Benzylalkohol, Natriumbenzoat, Chlorbutanol, Phenethylalkohol, Natriumbisulfit, Natriummetabisulfit und Tocopherol.

25. Verwendung einer Dosierungsform nach einem der Ansprüche 12 bis 24 bei der Herstellung eines Medikaments zum Reduzieren des Auftretens von Anfällen.

26. Verwendung nach Anspruch 25, wobei die Dosierungsform zusammen mit einem weiteren Anfälle hemmenden Arzneimittel verabreicht wird.

## Revendications

1. Une pluralité de particules de lamotrigine ayant une aire superficielle spécifique de deux à trois et demi mètres carrés par gramme.

2. La pluralité de particules de lamotrigine selon la revendication 1, ayant une aire superficielle spécifique d'environ trois mètres carrés par gramme.

3. La pluralité de particules de lamotrigine selon la revendication 1, dans laquelle le diamètre de toutes les particules de la pluralité est égal ou inférieur à 100 µm.

4. La pluralité de particules de lamotrigine selon la revendication 3, dans laquelle le diamètre de toutes les particules de la pluralité est égal ou inférieur à 50 µm.

5. La pluralité de particules de lamotrigine selon la revendication 4, dans laquelle le diamètre de toutes les particules de la pluralité est égal ou inférieur à 10 µm.

6. Une composition pharmaceutique comprenant une pluralité de particules de lamotrigine ayant une aire superficielle spécifique de deux à trois et demi mètres carrés par gramme.

7. La composition pharmaceutique selon la revendication 6 ayant une aire superficielle spécifique d'environ trois mètres carrés par gramme.

8. La composition pharmaceutique selon la revendication 6, dans laquelle le diamètre de toutes les particules de la pluralité est égal ou inférieur à 100 µm.

9. La composition pharmaceutique selon la revendication 8, dans laquelle le diamètre de toutes les particules de la pluralité est égal ou inférieur à 50 µm.

10. La composition pharmaceutique selon la revendication 9, dans laquelle le diamètre de toutes les particules de la pluralité est égal ou inférieur à 10 µm.

11. Une forme d'administration comprenant la composition pharmaceutique selon l'une quelconque des revendications 6 à 10.

12. La forme d'administration selon la revendication 11 qui est une forme d'administration solide à usage oral.

13. La forme d'administration solide à usage oral selon la revendication 12, dans laquelle la composition pharmaceutique comprend au moins un excipient acceptable du point de vue pharmaceutique.

14. La forme d'administration solide à usage oral selon la revendication 13, dans laquelle l'excipient acceptable du point de vue pharmaceutique est choisi dans le groupe consistant en cellulose microcristalline, cellulose microfine, lactose, amidon, amidon prégélatinisé, carbonate de calcium, sulfate de calcium, sucre, dextrates, dextrine, dextrose, phosphate de calcium dibasique dihydraté, phosphate de calcium tribasique, kaolin, carbonate de magnésium, oxyde de magnésium, maltodextrine, mannitol, polyméthacrylate, chlorure de potassium, cellulose pulvérulente, chlorure de sodium, sorbitol, talc, acacia, acide alginique, carbomère, carboxy méthylcellulose de sodium, dextrine, éthyl cellulose, gélatine, gomme guar, huile végétale hydrogénée, hydroxyéthyl cellulose, hydroxypropyl cellulose, hydroxypropyl méthylcellulose, glucose liquide, aluminosilicate de magnésium, maltodextrine, méthylcellulose, polyméthacrylates, povidone, amidon prégélatinisé, alginate de sodium, amidon, acide alginique, carboxyméthyl cellulose de calcium, dioxyde de silicium colloïdal, croscarmellose de sodium, crospovidone, gomme guar, aluminosilicate de magnésium, méthyl cellulose, polacriline de potassium, cellulose pulvérulente, amidon prégélatinisé, alginate de sodium et amidon glycolate de sodium.

15. La forme d'administration solide à usage oral selon l'une quelconque des revendications 12 à 14 contenant une dose unitaire d'environ 100 à environ 400 milligrammes de lamotrigine.

16. La forme d'administration selon la revendication 11, consistant en une forme d'administration liquide à usage oral.

17. La forme d'administration liquide à usage oral selon la revendication 16, dans laquelle la forme d'administration liquide à usage oral comprend un support liquide choisi dans le groupe consistant en eau, huile végétale, alcool, polyéthylène glycol, propylène glycol et glycérine.

18. La forme d'administration liquide à usage oral selon la revendication 17, dans laquelle le support liquide est l'eau.

19. La forme d'administration liquide à usage oral selon l'une quelconque des revendications 16 à 18, comprenant de plus au moins un excipient choisi dans le groupe consistant en gélatine, jaune d'oeuf, caséine, cholestérol, acacia, gomme adragante, carraghénane, pectine, méthyl cellulose, carbomère, alcool cétostéarylique, alcool cétylique, acide alginique, bentonite, carbomère, carboxyméthyl cellulose de calcium ou de sodium, éthylcellulose, gélatine, gomme guar, hydroxyéthyl cellulose, hydroxypropyl cellulose, hydroxypropyl méthylcellulose, maltodextrine, alcool polyvinylique, povidone, carbonate de propylène, alginate de propylène glycol, alginate de sodium, amidon glycolate de sodium, amidon adragante, gomme xanthane, sorbitol, saccharine, saccharine de sodium, sucrose, aspartame, fructose, mannitol, sucre inverti, alcool éthylique, benzoate de sodium, hydroxytoluène butylé, hydroxyanisole butylé, acide éthylènediamine tétraacétique, acide guconique, acide lactique, acide citrique, acide acétique, guconate de sodium, lactate de sodium, citrate de sodium et acétate de sodium.

20. La forme d'administration selon la revendication 11, consistant en une forme d'administration liquide à usage parentéral.

21. La forme d'administration liquide à usage parentéral selon la revendication 20, comprenant de plus un agent de modification de la tonicité.

22. La forme d'administration liquide à usage parentéral selon la revendication 21, dans laquelle l'agent de modification de la tonicité est le dextrose.

23. La forme d'administration liquide à usage parentéral selon la revendication 22, dans laquelle le dextrose est une solution à 5% de dextrose.

24. La forme d'administration liquide à usage parentéral selon l'une quelconque des revendications 20 à 23, comprenant de plus au moins un excipient choisi dans le groupe consistant en dextrose, glycérol, lactose, mannitol, sorbitol, acétate, citrate, tartrate, parabènes, phénols 1,6-dialkyl substitués, chlorure de benzalkonium, chlorure de benzéthonium, alcool benzylique, benzoate de sodium, chlorobutanol, alcool phénéthylique, bisulfite de sodium, métabisulfite de sodium et tocophérol.

25. L'utilisation d'une forme d'administration selon l'une quelconque des revendications 12 à 24, dans la fabrication d'un médicament destiné à réduire l'incidence d'attaques.

26. L'utilisation selon la revendication 25, dans laquelle la forme d'administration est administrée en association avec un autre médicament inhibant les attaques.
